# EUROPEAN PATENT SPECIFICATION

(11) **EP 1 553 938 B1**
(45) Date of publication and mention of the grant of the patent: **03.01.2007**
(21) Application number: 03748446.6
(22) Date of filing: 13.10.2003
(51) Int. Cl.: A61K 31/427, A61P 5/00, A61P 5/18, A61P 35/00

(54) **USE OF EPOTHILONE DERIVATIVES FOR THE TREATMENT OF HYPERPARATHYROIDISM**
VERWENDUNG VON EPOTHILONE ZUR BEHANDLUNG HYPERPARATHYREOIDISMUS
UTILISATION DE DERIVES D'EPOTHILONE POUR LE TRAITEMENT DE L'HYPERPARATHYROIDIE

(30) Priority: 15.10.2002 US 418592 P
(43) Date of publication of application: 20.07.2005
(73) Proprietor: BOARD OF SUPERVISORS OF LOUISIANA STATE UNIVERSITY AND AGRICULTURAL AND MECHANICAL COLLEGE, New Orleans, LA 70112 (US)
(72) Inventor: WOLTERING, Eugene, A., Kenner, LA 70065 (US)
(74) Representative: Dressel, Jürgen
(86) International application number: PCT/IB2003/004514
(87) International publication number: WO 2004/035050

(56) References cited:
- WO-A-98/22461
- WO-A-99/39694
- WO-A-99/67252

## Description

The present invention relates to a method of treating a warm-blooded animal, especially a human, having hyperparathyroidism comprising administering to said animal a therapeutically effective amount of an epothilone derivative of formula I as defined below.

The epothilones, especially epothilones A, B and D, represent a new class of microtubule stabilizing cytotoxic agents (see Gerth, K. et al., J. Antibiot. 49, 560-3 (1996); or Hoefle et al., DE 41 38 042).

Surprisingly, it was found that epothilone derivatives of formula I 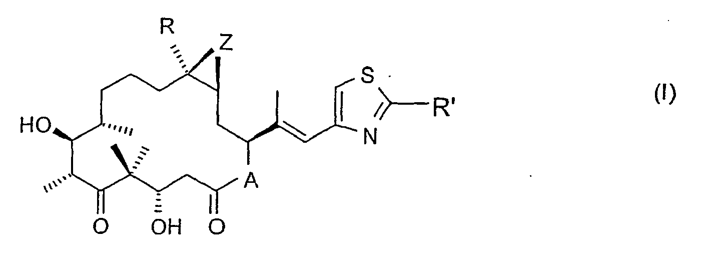 wherein A represents O or NR_{N}, wherein R_{N} is hydrogen or lower alkyl, R is hydrogen or lower alkyl, R' is methyl, methoxy, ethoxy, amino, methylamino, dimethylamino or methylthio, and Z is O or a bond, or a pharmaceutically acceptable salt thereof, produce a beneficial effect in the treatment of hyperparathyroidism.

Hence, the invention relates to a method of treating a warm-blooded animal having hyperparathyroidism comprising administering a therapeutically effective amount of an epothilone derivative of formula I wherein A represents O or NR_{N}, wherein R_{N} is hydrogen or lower alkyl, R is hydrogen or lower alkyl, R' is methyl, methoxy, ethoxy, amino, methylamino, dimethylamino or methylthio, and Z is O or a bond, or a pharmaceutically acceptable salt thereof to a warm-blooded animal in need thereof.

Unless stated otherwise, in the present disclosure organic radicals and compounds designated "lower" contain not more than 7, preferably not more than 4, carbon atoms.

A compound of formula I wherein A represents O, R is hydrogen and Z is O is known as epothilone A; a compound of formula I wherein A represents O, R is methyl and Z is O is known as epothilone B; a compound of formula I wherein A represents O, R is hydrogen and Z is a bond is known as epothilone C; a compound of formula I wherein A represents O, R is methyl and Z is a bond is known as epothilone D.

Epothilone derivatives of formula I wherein A represents O or NR_{N}, wherein R_{N} is hydrogen or lower alkyl, R is hydrogen or lower alkyl, R' is methyl and Z is O or a bond, and methods for the preparation of such epothilone derivatives are in particular generically and specifically disclosed in the patents and patent applications WO 93/10121, US 6,194,181, WO 98/25929, WO 98/08849, WO 99/43653, WO 98/22461 and WO 00/31247 in each case in particular in the compound claims and the final products of the working examples. Epothilone derivatives of formula I, especially epothilone B, can be administered in the form of pharmaceutical compositions which are disclosed in WO 99/39694.

Epothilone derivatives of formula I wherein A represents O or NR_{N}, wherein R_{N} is hydrogen or lower alkyl, R is hydrogen or lower alkyl, R' is methoxy, ethoxy, amino, methylamino, dimethylamino or methylthio, and Z is O or a bond, and methods for the preparation and administration of such epothilone derivatives are in particular generically and specifically disclosed in the patent application WO99/67252.

The transformation of epothilone B to the corresponding lactam is disclosed in Scheme 21 (page 31, 32) and Example 3 of WO 99/02514 (pages 48 - 50). The transformation of a compound of formula I which is different from epothilone B into the corresponding lactam can be accomplished analogously. Corresponding epothilone derivatives of formula I wherein R_{N} is lower alkyl can be prepared by methods known in the art such as a reductive alkylation reaction starting from the epothilone derivative wherein R_{N} is hydrogen.

The terms "treatment" or "treating" as used herein comprises the treatment of patients having hyperparathyroidism or being in a pre-stage of said disease which treatment produces one or more of the following effects in hyperparathyroidism patients:
- a reduction in parathyroid hormone levels in blood,
- a reduction in parathyroid hormone levels in urine,
- a reduction of calcium levels in blood,
- a reduction of calcium levels in urine,
- an increase in bone density.

It will be understood that in the discussion of methods, references to the active ingredients are meant to also include the pharmaceutically acceptable salts. If these active ingredients have, for example, at least one basic center, they can form acid addition salts. Corresponding acid addition salts can also be formed having, if desired, an additionally present basic center. The active ingredients having an acid group (for example COOH) can also form salts with bases. The active ingredient or a pharmaceutically acceptable salt thereof may also be used in form of a hydrate or include other solvents used for crystallization.

In one preferred embodiment of the invention, an epothilone derivative of formula I is employed wherein A represents O, R is lower alkyl, especially methyl, ethyl or n-propyl, or hydrogen and Z is O or a bond. More preferably, an epothilone derivative of formula I is employed wherein A represents O, R is methyl and Z is O, which compound is also known as epothilone B.

Throughout the present specification and claims hyperparathyroidism means primary, secondary or tertiary hyperparathyroidism. Furthermore, three different forms of tertiary hyperparathyroidism are known, which are adenoma, hyperplasia and carcinoma.

Control of hypercalcemia from recurrent or persistent parathyroid adenoma, parathyroid hyperplasia, or from parathyroid carcinoma is difficult, and on occasion impossible. A number of approaches have been used to control the symptomatic hypercalcemia induced by these conditions.

The present invention pertains preferably to parathyroid adenoma, parathyroid hyperplasia and parathyroid carcinoma, more preferably, to recurrent or persistent parathyroid adenoma, parathyroid hyperplasia and parathyroid carcinoma.

The symptoms induced by hypercalcemia life can be threatening. This is particularly true of parathyroid carcinoma, where patients typically die from uncontrolled hypercalcemia.

One embodiment the present invention pertains to the control of hypercalcemia resulting from parathyroid adenoma, parathyroid hyperplasia and parathyroid carcinoma.

The method of treating a warm-blooded animal having hyperparathyroidism as disclosed herein can be employed as a monotherapy or in addition to an established therapy comprising, e.g., the administration of a standard anti-diarrheal.

The term "standard anti-diarrheal" as used herein include, natural opiods, such as tincture of opium, paregoric, and codeine, synthetic opoids, such as diphenoxylate, difenoxin and loperamide, bismuth subsalicylate, octreotide, motilin antagonists and traditional antidiarrheal remedies, such as kaolin, pectin, berberine and muscarinic agents. The antidiarrheal agent is administered as a preventative measure throughout the treatment cycle or as needed when diarrhea occurs. The antidiarrheal agent is administered to prevent, control or eliminate diarrhea that is sometimes associated with the administration of epothilones, especially epothilone B.

The structure of the active ingredients identified by code nos., generic or trade names may be taken from the actual edition of the standard compendium "The Merck Index" or from databases, e.g. Patents International (e.g. IMS World Publications).

The pharmacological activity of a compound of formula I, in particular epothilone B, can be demonstrated, e.g., in a study wherein patients suffering from hyperparathyroidism are treated with continuous 4-week cycles (three weeks on/one week off) of epothilone B until either disease progression or unacceptable side effects occur. Response initially can be evaluated after the first two cycles, and can be based on unchanged or improvement in clinical symptoms. Evaluations for response can be performed, e.g., every two cycles thereafter.

It is submitted that at least part of the effect observed with the compounds of formula I on the diseases mentioned herein are resulting from the inhibition of angiogenesis. A number of *in vitro* angiogenesis assays exist, however, they do not provide potentially useful information for the treatment of an individual patient. Clonogenic assays have been utilized to evaluate the response of an individual's tumor to antineoplastic agents, but these tumor fragments are cultured in an environment that does not lead to neovessel growth. It is known that human vein disks incorporated into a 0.3% fibrin-thrombin clot will develop angiogenic vessel growth from the cut edge of the vessel disk. The *in vitro* angiogenesis assay described below can be employed to demonstrate the utility of the compounds of formula I for the treatment of the diseases mentioned herein.

METHODS: Tumor disks (2mm in diameter) from seven fresh surgical specimens are incorporated into fibrin-thrombin clots overlayed with nutrient medium containing 20% fetal bovine serum. The fragments are allowed to become angiogenic and on day 18, nutrient medium or nutrient medium containing a compound of formula I, is added .Tumor disks are visually assessed over time to determine the percent of wells that initiated an angiogenic response (% I). Neovessel growth, density, and length are graded at intervals using a semiquantitative visual neovessel growth-rating scheme [angiogenic index (Al), 0-16 scale]. Statistical significance of the results is tested using comparison of two proportions for % initiation and t-tests for the angiogenic index (p< .05 considered significant*).

Pharmaceutical composition suitable for the treatment of hyperparathyroidism contain, for example, from 10 % to 100 %, preferably from 20 % to 60 %, of the active ingredients of formula I. Pharmaceutical preparations for enteral or parenteral administration are, for example, those in unit dosage forms, such as sugar-coated tablets, tablets, capsules or suppositories, and furthermore ampoules. If not indicated otherwise, these are prepared in a manner known per se.

The effective dosage of a compound of formula I may vary depending on the particular compound or pharmaceutical composition employed, the mode of administration, the severity of the hyperparathyroidism being treated. Thus, the dosage of a compound of formula I is selected in accordance with a variety of factors including the route of administration and the renal and hepatic function of the patient. A physician, clinician or veterinarian of ordinary skill can readily determine and prescribe the effective amount of a compound of formula I required to prevent, counter or arrest the progress of the condition. Optimal precision in achieving concentration of the active ingredients within the range that yields efficacy without toxicity requires a regimen based on the kinetics of the active ingredients' availability to target sites.

If the the warm-blooded animal is a human, the dosage of a compound of formula I is preferably in the range of about 0.1 to 75, preferably 0.25 to 50, e.g. 2.5 or 6, mg/m² once weekly for two to four, e.g. three, weeks, followed by 6 to 8 days off in the case of an adult patient.

In one embodiment of the invention epothilone B is administered weekly in a dose that is between about 0.1 to 6 mg/m², preferably between 0.1 and 3 mg/m², e.g. 2.5 mg/m², for three weeks after an interval of one to six weeks, especially an interval of one week, after the preceding treatment. In another embodiment of the invention said epothilone B is preferably administered to a human every 18 to 24 days in a dose that is between 0.5 and 7.5 mg/m².

In another embodiment of the invention, epothilone B is provided continuously, e.g. for a week or four weeks, at a dose that effects a blood level of 10 to 12 M or higher. Peferably, the drug is applied in that embodiment by means of a sustained release formulation.

The invention also provides the use of a compound of formula I for the preparation of a medicament for the treatment of hyperparathyroidism or of the diseases mentioned herein.

### EXAMPLE 1

The following results were obtained with epothilone B in the *in vitro* angiogenesis assay

| **Tumor Type** | **% Initiation Control EpoB 10⁻⁸ M** | | **% ↓** | **P <.05** | **Al (0-16) Control EpoB 10⁻⁸ M** | | **% ↓** | **P <.05** |
|---|---|---|---|---|---|---|---|---|
| Breast carcinoma | 55.2 | 3.3 | 94 | * | 10.6 | 1 | 91 | * |
| Bronchial carcinoid | 41.7 | 37 | 12 | NS | 10.1 | 6.1 | 40 | NS |
| Midgut carcinoid | 23.3 | 20 | 15 | NS | 13.7 | 8.2 | 40 | * |
| Midgut carcinoid (met) | 41.4 | 0 | 100 | * | 8.6 | 0 | 100 | * |
| Thyroid cancer | 100 | 50 | 50 | * | 14.1 | 2.1 | 85 | * |
| Renal cell carcinoma | 60 | 6.7 | 88 | * | 4.9 | 1 | 80 | * |
| Thymic carcinoid | 8.3 | 3.3 | 40 | NS | 9.2 | 2.5 | 73 | NS |
| **Mean +/- SEM** | **47.1± 11.1** | **17.2± 7.4** | **60± 14** | **-** | **10.2± 1.2** | **3± 1.1** | **73± 9** | **-** |

RESULTS: All tumors initiated an angiogenic response *in vitro.* The mean percent of wells that initiated an angiogenic response was 47.1 ± 11.1 % in this group of tumors. A thymic carcinoid was the least angiogenic (8.3% of wells initiated), while a thyroid cancer, which was identified as a parathyroid adenoma, exhibited 100% initiation. Following initiation, tumors exhibited progressive increases in neovessel growth, length, and density over time. The mean angiogenic index of these tumors was 10.2 ± 1.2. Treatment of these tumors with 10⁻⁸ M epothilone B significantly decreased (60 ± 13.8%) the initiation of their angiogenic response. Subsequent vessel development was also significantly inhibited (73 ± 9%) by epothilone B treatment.

CONCLUSIONS: Epothilone B may be an effective antiangiogenic agent in a variety of tumor types. The employed *in vitro* model provides useful information to the clinician on the effect of specific antiangiogenic agents on an individual patient's tumor. This may be particularly useful in patients with tumors that, as a group, are unresponsive to treatment with antineoplastic agents.

## Claims

1. The use of a compound of formula I 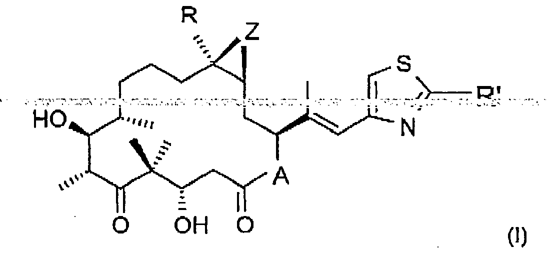 wherein A represents O or NR_{N}, wherein R_{N} is hydrogen or lower alkyl, R is hydrogen or lower alkyl, R' is methyl, methoxy, ethoxy, amino, methylamino, dimethylamino or methylthio, and Z is O or a bond, or a pharmaceutically acceptable salt thereof, for the preparation of a medicament for the treatment of hyperparathyroidism, in warm-blooded animals, wherein "lower alkyl" means not move than 7 carbon atom.

2. The use of a compound according to claim 1 where in A represents O, R is methyl and Z is O or a pharmaceutically acceptable salt thereof.

3. The use according to claim 1 wherein treatment comprises administering said epothilone derivative weekly in a dose that is between about 0.1 to 6 mg/m² for three weeks after an interval of one to six weeks after the preceding treatment.

4. The use according to any one of claims 1 to 3 wherein the hyperparathyroidism disease is adenoma, hyperplasia or carcinoma.

5. The use according to claim 4 wherein the disease is parathyroid adenoma, parathyroid hyperplasia or parathyroid carcinoma.

6. The use according to any one of claims 4 or 5 wherein the parathyroid cancer disease is recurrent or persistent parathyroid adenoma, recurrent or persistent parathyroid hyperplasia or recurrent or persistent parathyroid carcinoma.

7. The use according to any one of claims 1 to 3 wherein the hyperparathyroidism disease is primary or secondary hyperparathyroidism.

8. The use of a compound of formula I 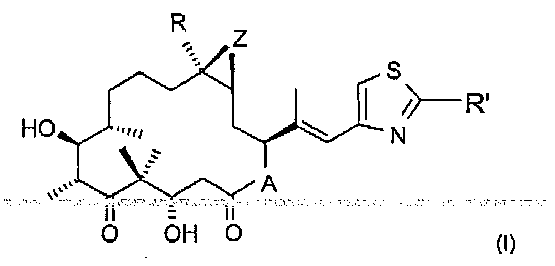 wherein A represents O or NR_{N}, wherein R_{N} is hydrogen or lower alkyl, R is hydrogen or lower alkyl, R' is methyl, methoxy, ethoxy, amino, methylamino, dimethylamino or methylthio, and Z is O or a bond, or a pharmaceutically acceptable salt thereof, for the preparation of a medicament for the treatment of hypercalcemia resulting from parathyroid adenoma, parathyroid hyperplasia or parathyroid carcinoma in warm-blooded animals, wherein "lower alkyl" means not more than 7 carbon atoms.

9. The use of a compound according to claim 8 wherein A represents O, R is methyl and Z is O or a pharmaceutically acceptable salt thereof.

10. The use according to claim 9 wherein treatment comprises administering said epothilone derivative weekly in a dose that is between about 0.1 to 6 mg/m² for three weeks after an interval of one to six weeks after the preceding treatment

11. The use according to to any one of claims 8 to 10 wherein the disease is recurrent or persistent parathyroid adenoma, recurrent or persistent parathyroid hyperplasia or recurrent or persistent parathyroid carcinoma.

## Patentansprüche

1. Verwendung einer Verbindung der Formel I 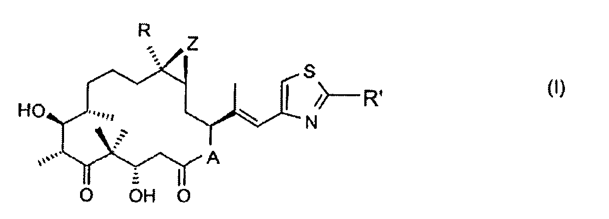 worin A für O oder NR_{N} steht, worin R_{N} für Wasserstoff oder Niederalkyl steht, R für Wasserstoff oder Niederalkyl steht, R' für Methyl, Methoxy, Ethoxy, Amino, Methylamino, Dimethylamino oder Methylthio steht und Z für O oder eine Bindung steht, oder eines pharmazeutisch annehmbaren Salzes hiervon zur Herstellung eines Arzneimittels für die Behandlung von Hyperparathyreoidismus bei Warmblütern, worin Niederalkyl nicht mehr als 7 Kohlenstoffatome bedeutet.

2. Verwendung einer Verbindung nach Anspruch 1, worin A für O steht, R für Methyl steht und Z für O steht, oder eines pharmazeutisch annehmbaren Salzes hiervon.

3. Verwendung nach Anspruch 1, worin die Behandlung eine wöchentliche Verabreichung des Epothilonderivats in einer Dosis umfasst, die zwischen etwa 0,1 und 6 mg/m² liegt, während drei Wochen nach einem Intervall von einer bis sechs Wochen nach der vorhergehenden Behandlung.

4. Verwendung nach einem der Ansprüche 1 bis 3, worin die Hyperparathyreoidismuskrankheit ein Adenom, eine Hyperplasie oder ein Carcinom ist.

5. Verwendung nach Anspruch 1, worin die Krankheit ein Parathyreoidadenom, eine Parathyreoidhyperplasie oder ein Parathyreoidcarcinom ist.

6. Verwendung nach einem der Ansprüche 4 oder 5, worin die Parathyreoidkrebskrankheit ein wiederkehrendes oder persistentes Parathyreoidadenom, eine wiederkehrende oder persistente Parathyreoidhyperplasie oder ein wiederkehrendes oder persistentes Parathyreoidcarcinom ist.

7. Verwendung nach einem der Ansprüche 1 bis 3, worin die Hyperparathyreoidismuskrankheit ein primärer oder sekundäre Hyperparathyreoidismus ist.

8. Verwendung einer Verbindung der Formel I 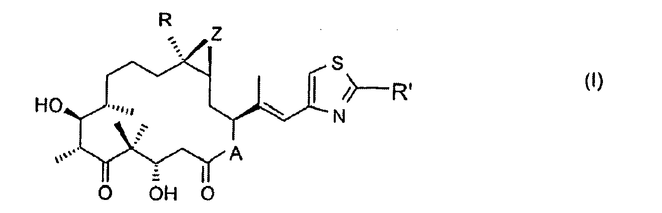 worin A für O oder NR_{N} steht, worin R_{N} für Wasserstoff oder Niederalkyl steht, R für Wasserstoff oder Niederalkyl steht, R' für Methyl, Methoxy, Ethoxy, Amino, Methylamino, Dimethylamino oder Methylthio steht und Z für O oder eine Bindung steht, oder eines pharmazeutisch annehmbaren Salzes hiervon, zur Herstellung eines Arzneimittels für die Behandlung von Hypercalcämie infolge eines Parathyreoidadenoms, einer Parathyreoidhyperplasie oder eines Parathyreoidcarcinoms bei Warmblütern, worin Niederalkyl nicht mehr als 7 Kohlenstoffatome bedeutet.

9. Verwendung einer Verbindung nach Anspruch 8, worin A für O steht, R für Methyl steht und Z für O steht, oder eines pharmazeutisch annehmbaren Salzes hiervon.

10. Verwendung nach Anspruch 9, worin die Behandlung eine wöchentliche Verabreichung des Epothilonderivats in einer Dosis umfasst, die zwischen etwa 0,1 und 6 mg/m² liegt, während drei Wochen nach einem Intervall von einer bis sechs Wochen nach der vorhergehenden Behandlung.

11. Verwendung nach einem der Ansprüche 8 bis 10, worin die Krankheit ein wiederkehrendes oder persistentes Parathyreoidadenom, eine wiederkehrende oder persistente Parathyreoidhyperplasie oder ein wiederkehrendes oder persistentes Parathyreoidcarcinom ist.

## Revendications

1. Utilisation d'un composé de formule I 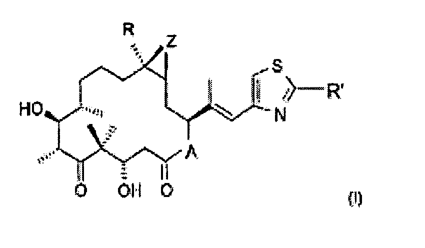 dans laquelle A représente O ou NR_{N}, où R_{N} est un atome d'hydrogène ou un groupe alkyle inférieur, R est un atome d'hydrogène ou un groupe alkyle inférieur, R' est un groupe méthyle , méthoxy, éthoxy, amino, méthylamino, diméthylamino ou méthylthio, et Z est O ou une liaison, ou un sel pharmaceutiquement acceptable de celui-ci, pour la préparation d'un médicament pour le traitement de l'hyperparathyoïdie chez des animaux à sang chaud, où « groupe alkyle inférieur » signifie pas plus de 7 atomes de carbone.

2. Utilisation d'un composé selon la revendication 1, dans laquelle A représente O, R est un groupe méthyle et Z est O ou un sel pharmaceutiquement acceptable de celui-ci

3. Utilisation d'un composé selon la revendication 1, dans laquelle le traitement comprend l'administration hebdomadaire dudit dérivé d'épothilone sous la forme d'une dose comprise entre 0,1 et 6 mg/m² pendant trois semaines après un intervalle d'une à six semaines après le traitement précédent.

4. Utilisation selon l'une quelconque des revendications 1 à 3, dans laquelle l'hyperparathyroïdie est l'adénome, l'hyperplasie ou le carcinome.

5. Utilisation selon la revendication 4, dans laquelle la maladie est l'adénome de la parathyroïde, l'hyperplasie de la parathyroïde ou le carcinome de la parathyroïde.

6. Utilisation selon l'une quelconque des revendications 4 ou 5, dans laquelle le cancer de la parathyroïde est l'adénome de la parathyroïde récurrent ou persistant, l'hyperplasie de la parathyroïde récurrente ou persistante ou le carcinome de la parathyroïde récurrent ou persistant.

7. Utilisation selon l'une quelconque des revendications 1 à 3, dans laquelle l'hyperparathyroïdie est l'hyperparathyroïdie primaire ou secondaire.

8. Utilisation d'un composé de formule 1 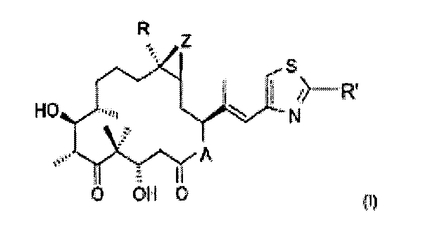 dans laquelle A représente O ou NR_{N}, où R_{N} est un atome d'hydrogène ou un groupe alkyle inférieur, R est un atome d'hydrogène ou un groupe alkyle inférieur, R' est un groupe méthyle , méthoxy, éthoxy, amino, méthylamino, diméthylamino ou méthylthio, et Z est O ou une liaison, ou un sel pharmaceutiquement acceptable de celui-ci, pour la préparation d'un médicament pour le traitement de l'hypercalcémie résultant de l'adénome de la parathyroïde, de l'hyperplasie de la parathyroïde ou du carcinome de la parathyroïde chez des animaux à sang chaud, où « groupe alkyle inférieur » signifie pas plus de 7 atomes de carbone.

9. Utilisation d'un composé selon la revendication 8, dans laquelle A représente O, R est un groupe méthyle et Z est O ou un sel pharmaceutiquement acceptable de celui-ci.

10. Utilisation selon la revendication 9, dans laquelle le traitement comprend l'administration hebdomadaire dudit dérivé d'épothilone sous la forme d'une dose comprise entre 0,1 et 6 mg/m² pendant trois semaines après un intervalle d'une à six semaines après le traitement précédent.

11. Utilisation selon l'une quelconque des revendications 8 à 10, dans laquelle la maladie est l'adénome de la parathyroïde récurrent ou persistant, l'hyperplasie de la parathyroïde récurrente ou persistante ou le carcinome de la parathyroïde récurrent ou persistant.
